# EUROPEAN PATENT APPLICATION

(11) **EP 4 119 125 A1**
(43) Date of publication of application: **18.01.2023**
(21) Application number: 22168121.6
(22) Date of filing: 13.04.2022
(51) Int. Cl.: A61K 9/08, A61L 12/14, C11D 3/00

(54) **LIQUID COMPOSITION FOR OPHTHALMIC PRODUCT**

(30) Priority: 16.07.2021 TW 110126330
(71) Applicant: Pegavision Corporation, Taoyuan City 333 (TW)
(72) Inventor: CHANG, Hung-Ju, 333 TAOYUAN CITY (TW); TING, Wei-Jia, 333 TAOYUAN CITY (TW); HUANG, Chi-Hao, 333 TAOYUAN CITY (TW); WU, Hsin-Yi, 333 TAOYUAN CITY (TW)
(74) Representative: Winter, Brandl - Partnerschaft mbB

(57) **Abstract**

Provided is a liquid composition for ophthalmic products. The liquid composition includes a buffer solution and a zinc salt dissolved in the buffer solution. The zinc salt has a weight percentage concentration of 1×10⁻⁵% to 3% in the liquid composition. The liquid composition may also include a first vitamin dissolved in the buffer solution. The first vitamin has a weight percentage concentration of 1×10⁻⁵% to 1% in the liquid composition. The first vitamin includes vitamin B2, a vitamin B2 derivative, or a combination thereof.

## Description

### BACKGROUND

### Field of Invention

The disclosure relates to a liquid composition used in ophthalmic products. The liquid composition enhances user comfort and keeps the cornea healthy when wearing contact lenses.

### Description of Related Art

With the advent of the information society, the frequent use of 3C products, for example, smartphones or computers, has led to an increasing proportion of myopia. The age group of myopia has also gradually declined. Considering the beauty and convenience, wearing contact lenses has gradually become a popular trend.

Since most people with poor vision have the habit of wearing contact lenses for a long time, the comfort of wearing contact lenses is very important. When wearing contact lenses, the oxygen permeability, water retention, and lubrication of the lenses are important indicators that determine comfort.

The oxygen permeability, water retention, and lubrication of the lenses have been improved with the improvement of the material for contact lenses. However, with the increase in the time of wearing contact lenses, the eyes of wearer, depending on the wearing environment and the healthy state of the eyes, are prone to discomfort due to lack of oxygen and water, especially in the cold air room for a long time, and even cause corneal injuries and lesions.

Usually, the addition of moisturizers such as glycerol to contact lens preservation solutions can only alleviate the discomfort of wearing contact lenses. Therefore, there is an urgent need for a novel ophthalmic formula to work with contact lenses in improving the comfort of wearing contact lenses and keeping the cornea healthy.

### SUMMARY

An aspect of the present disclosure provides a liquid composition for ophthalmic products. In some embodiments of the present disclosure, the liquid composition may be used as a preservation solution for contact lenses, i.e., the user may place the contact lenses in the liquid composition for preservation when not wearing contact lenses. The liquid composition includes a buffer solution and a zinc salt. The zinc salt is dissolved in the buffer solution, and a weight percentage concentration of the zinc salt in the liquid composition is 1×10⁻⁵% to 3%.

In one or more embodiments of the present disclosure, the zinc salt includes zinc sulfate, zinc sulfate hydrate, zinc gluconate, zinc lactate, zinc chloride, zinc citrate, chelated zinc, zinc acetate, or combinations thereof.

In one or more embodiments of the present disclosure, the buffer solution includes borate, phosphate, or a combination thereof.

In one or more embodiments of the present disclosure, the liquid composition further includes a hydrophilic substance, a surfactant, a vitamin, or combinations thereof.

In one or more embodiments of the present disclosure, the vitamin includes vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof.

In one or more embodiments of the present disclosure, the vitamin has a weight percentage concentration of 1×10⁻⁵% to 5% in the liquid composition.

In one or more embodiments of the present disclosure, the hydrophilic substance has a weight percentage concentration of 1×10⁻³% to 3% in the liquid composition.

In one or more embodiments of the present disclosure, the surfactant has a weight percentage concentration of 1×10⁻³% to 2% in the liquid composition.

Another aspect of the present disclosure provides a liquid composition for ophthalmic products, including a buffer solution and a first vitamin dissolved in the buffer solution. The first vitamin has a weight percentage concentration of 1×10⁻⁵% to 1% in the liquid composition, and the first vitamin includes vitamin B2, vitamin B2 derivative, or combinations thereof.

In one or more embodiments of the present disclosure, the first vitamin includes riboflavin, flavin mononucleotide and its salt, flavin adenine dinucleotide and its salt, or combinations thereof.

In one or more embodiments of the present disclosure, the buffer solution includes borate and phosphate.

In one or more embodiments of the present disclosure, the liquid composition further includes a hydrophilic substance, a surfactant, a second vitamin, or combinations thereof.

In one or more embodiments of the present disclosure, the second vitamin includes vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof.

In one or more embodiments of the present disclosure, the second vitamin has a weight percentage concentration of 1×10⁻⁵% to 5% in the liquid composition.

In one or more embodiments of the present disclosure, the hydrophilic substance has a weight percentage concentration of 1×10⁻³% to 3% in the liquid composition.

In one or more embodiments of the present disclosure, the surfactant has a weight percentage concentration of 1×10⁻³% to 2% in the liquid composition.

Yet another aspect of the present disclosure provides a liquid composition for ophthalmic products, including a buffer solution and a zinc salt and a first vitamin dissolved in the buffer solution. The zinc salt has a weight percentage concentration of 1×10⁻⁵% to 3% in the liquid composition. The first vitamin has a weight percentage concentration of 1×10⁻⁵% to 1% in the liquid composition, and the first vitamin includes vitamin B2, vitamin B2 derivative, or a combination thereof.

In one or more embodiments of the present disclosure, the weight percentage concentration of the zinc salt is between 5×10⁻⁵% to 2%.

In one or more embodiments of the present disclosure, the zinc salt includes zinc sulfate, zinc sulfate hydrate, zinc gluconate, zinc lactate, zinc chloride, zinc citrate, chelated zinc, zinc acetate, or combinations thereof.

In one or more embodiments of the present disclosure, the weight percentage concentration of the first vitamin is between 5×10⁻⁵% to 0.5%.

In one or more embodiments of the present disclosure, the first vitamin includes riboflavin, flavin mononucleotide and its salt, flavin adenine dinucleotide and its salt, or combinations thereof.

In one or more embodiments of the present disclosure, the buffer solution includes borate, phosphate, or a combination thereof.

In one or more embodiments of the present disclosure, the buffer solution further includes boric acid, sodium chloride, or a combination thereof.

In one or more embodiments of the present disclosure, the liquid composition further includes a hydrophilic substance, a surfactant, a second vitamin, or combinations thereof.

In one or more embodiments of the present disclosure, the second vitamin includes vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof.

In one or more embodiments of the present disclosure, the second vitamin has a weight percentage concentration of 1×10⁻⁵% to 5% in the liquid composition.

In one or more embodiments of the present disclosure, the weight percentage concentration of the second vitamin is between 5×10⁻⁵% to 3%.

In one or more embodiments of the present disclosure, the hydrophilic substance has a weight percentage concentration of 1×10⁻³% to 3% in the liquid composition.

In one or more embodiments of the present disclosure, the weight percentage concentration of the hydrophilic substance is between 5×10⁻³% to 2.5%.

In one or more embodiments of the present disclosure, the surfactant has a weight percentage concentration of 1×10⁻³% to 2% in the liquid composition.

In one or more embodiments of the present disclosure, the weight percentage concentration of the surfactant is between 5×10⁻³% to 1%.

### DETAILED DESCRIPTION

An aspect of the present disclosure provides a liquid composition for ophthalmic products. In some embodiments of the present disclosure, the liquid composition may be used as a preservation solution for contact lenses, i.e., the user may place the contact lens in this liquid composition for preservation when not wearing contact lenses.

The liquid composition includes a buffer solution and a zinc salt. In one or more embodiments of the present disclosure, the buffer solution includes borate and phosphate. The borate may be, for example, but not limited to borax. The phosphate may be, for example, but not limited to monosodium phosphate, sodium phosphate, monopotassium phosphate, and dipotassium phosphate. In one or more embodiments of the present disclosure, the buffer solution further includes but not limited to boric acid, sodium chloride, or a combination thereof.

The zinc salt is dissolved in the buffer solution and releases zinc ions in the buffer solution. In one or more embodiments of the present disclosure, the zinc salt includes but not limited to zinc sulfate, zinc sulfate hydrate, zinc gluconate, zinc lactate, zinc chloride, zinc citrate, chelated zinc, zinc acetate, or combinations thereof. The zinc salt has a weight percentage concentration of 1×10⁻⁵% to 3% in the liquid composition, preferably 5×10⁻⁵% to 2%, e.g., 5×10⁻⁴%, 1×10⁻⁴%, 5×10⁻³%, 1×10⁻³%, 0.05%, 0.01%, 0.5%, or 0.1%. It is worth noting that when the weight percentage concentration of the zinc salt in the liquid composition is in the above range or value, it will have unexpected technical effects described more later.

In one or more embodiments of the present disclosure, the liquid composition further includes a hydrophilic substance, a surfactant, a vitamin, or combinations thereof. In one or more embodiments of the present disclosure, the hydrophilic substance includes but not limited to polyvinyl alcohol, cellulose, cellulose derivative, polyvinylpyrrolidone, hyaluronic acid, polyglutamic acid, 2-methacryloyloxyethyl phosphorylcholine, polymers of phosphorylcholine, polyethylene glycol, or combinations thereof. In one or more embodiments of the present disclosure, the hydrophilic substance has a weight percentage concentration of 1×10⁻³% to 3% in the liquid composition, preferably 5×10⁻³% to 2.5%, e.g., 0.01%, 0.05%, 0.1%, 0.5%, 1%, 1.5%, or 2%.

In one or more embodiments of the present disclosure, the surfactant includes but not limited to polyoxyethylene (20) sorbitan monolaurate (Tween 20), polyoxyethylene sorbitan monostearate (Tween 60), polyoxyethylene sorbitan monooleate (Tween 80), sodium lauroyl lactylate, polyoxypropylene glycol, polyoxyethylene hardened castor oil, sodium dodecyl sulfate, GEROPON^{®} SBFA-30, or combinations thereof. In one or more embodiments of the present disclosure, the surfactant has a weight percentage concentration of 1×10⁻³% to 2% in the liquid composition, preferably 5×10⁻³% to 1%, e.g., 0.01%, 0.05%, or 0.5%.

In one or more embodiments of the present disclosure, the vitamin includes vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof. In one or more embodiments of the present disclosure, the vitamin has a weight percentage concentration of 1×10⁻⁵% to 5% in the liquid composition, preferably 5×10⁻⁵% to 3%, e.g., 1×10⁻⁴%, 5×10⁻⁴%, 1×10⁻³%, 5×10⁻³%, 0.01%, 0.05%, 0.1%, 0. 5%, 1%, or 2%. It is worth noting that when the weight percentage concentration of the above vitamin in the liquid composition is in the above range or value, it will have unexpected technical effects described more later.

Another aspect of the present disclosure provides a liquid composition for ophthalmic products, including a buffer solution and a first vitamin dissolved in the buffer solution. In one or more embodiments of the present disclosure, the buffer solution includes borate, phosphate, or a combination thereof. Borate and phosphate materials have been provided as described above and will not be repeated herein. In one or more embodiments of the present disclosure, the buffer solution further includes but not limited to boric acid, sodium chloride, or a combination thereof.

The first vitamin includes vitamin B2, vitamin B2 derivative, or a combination thereof. The structure of vitamin B2 derivative may be indicated by formula (I): 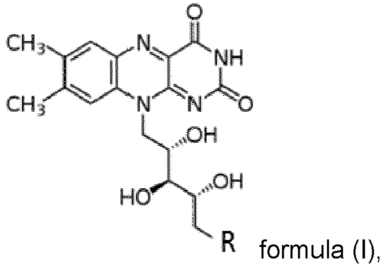 where R is a hydroxyl group, a phosphate group, and a derivative including phosphate. Vitamin B2 derivative may be, for example, but not limited to flavin mononucleotide and flavin adenine dinucleotide. In one or more embodiments of the present disclosure, the first vitamin includes riboflavin, flavin mononucleotide and its salts, flavin adenine dinucleotide and its salts, or combinations thereof. The first vitamin has a weight percentage concentration of 1×10⁻⁵% to 1% in the liquid composition, preferably 5×10⁻⁵% to 0.5%, e.g., 1×10⁻⁴%, 5×10⁻⁴%, 1×10⁻³%, 5×10⁻³%, 0.01%, 0.05%, or 0.1%. It is worth noting that when the weight percentage concentration of the first vitamin in the liquid composition is in the above range or value, it will have unexpected technical effects described more later.

In one or more embodiments of the present disclosure, the liquid composition further includes a hydrophilic substance, a surfactant, a second vitamin, or combinations thereof. The materials and weight percentage concentrations of the hydrophilic substance and the surfactant in the liquid composition have been provided as described above and will not be repeated herein.

In one or more embodiments of the present disclosure, the second vitamin includes vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof. In one or more embodiments of the present disclosure, the second vitamin has a weight percentage concentration of 1×10⁻⁵% to 5% in the liquid composition, preferably 5×10⁻⁵% to 3%, e.g., 1×10⁻⁴%, 5×10⁻⁴%, 1×10⁻³%, 5×10⁻³%, 0.01%, 0.05%, 0.1%, 0.5%, 1%, or 2%. It is worth noting that when the weight percentage concentration of the second vitamin in the liquid composition is in the above range or value, it will have unexpected technical effects described more later.

Yet another aspect of the present disclosure provides a liquid composition for ophthalmic products, including a buffer solution and a zinc salt and a first vitamin dissolved in the buffer solution. In one or more embodiments of the present disclosure, the buffer solution includes borate, phosphate, or a combination thereof. Borate and phosphate materials have been provided as described above and will not be repeated herein. In one or more embodiments of the present disclosure, the buffer solution further includes but not limited to boric acid, sodium chloride, or a combination thereof.

The zinc salt is dissolved in the buffer solution and releases zinc ions in the buffer solution. In one or more embodiments of the present disclosure, the material of the zinc salt and its weight percentage concentration in the liquid composition have been provided as described above and will not be repeated herein. It is worth noting that when the weight percentage concentration of the zinc salt in the liquid composition is in the above range or value, it will have unexpected technical effects described more later.

The first vitamin includes vitamin B2, vitamin B2 derivative, or a combination thereof. The structures of vitamin B2 and vitamin B2 derivative and their weight percentage concentrations in the liquid composition have been provided as described above, which will not be repeated herein. It is worth noting that when the weight percentage concentration of the first vitamin in the liquid composition is in the above range or value, it will have unexpected technical effects described more later.

In one or more embodiments of the present disclosure, the liquid composition further includes a hydrophilic substance, a surfactant, a second vitamin, or combinations thereof. The hydrophilic substance, surfactant and second vitamin materials and their individual weight percentage concentrations in the liquid composition have been provided as described above, which will not be repeated herein.

Subsequently, three embodiments and one comparative embodiment are provided. Embodiments are provided for a detailed illustration of specific embodiments of the present disclosure so that those skilled in the art can implement the present disclosure. It should not be explained as a limitation of the disclosure.

In the embodiments and comparative embodiments, the subjects wore the same type of contact lenses. The main difference between the embodiments and the comparative embodiments was that the contact lenses of the embodiments were pre-stored in the liquid composition of the present disclosure, while the contact lenses of the comparative embodiments were pre-stored in other liquid composition. It was worth noting that the liquid composition of the present disclosure included one of the zinc salt, vitamin B2, vitamin B2 derivative, or combinations thereof. However, the liquid composition pre-stored the contact lenses in the comparative embodiments did not include any of the zinc salt, vitamin B2, and vitamin B2 derivative.

The first set of embodiments included Embodiment A1 to Embodiment A12. Table 1 records the specific components of Embodiment A1 to Embodiment A12 and their weight percentage concentrations (unit in %) in the liquid composition, which may include:
1. Buffer solution: the buffer solution simultaneously including borax, boric acid, and sodium chloride
2. Hydrophilic substance: hyaluronic acid and polyvinyl alcohol (PEG 400) with an average molecular weight of 400
3. Surfactant: polyoxyethylene sorbitan monooleate (Tween 80), which had a weight percentage concentration of 0.08% in the liquid composition
4. Zinc salt: zinc sulfate, zinc lactate
5. Vitamin: vitamin B6, vitamin B12, vitamin E

In each Embodiment A1 to Embodiment A3, the liquid composition included one of zinc sulfate or zinc lactate, and zinc sulfate or zinc lactate in the liquid composition by weight percentage concentration of 0.001%. Further, Embodiment A1 did not include hyaluronic acid, PEG 400, and TWEEN 80. Embodiment A2 and Embodiment A3 both simultaneously included hyaluronic acid, PEG 400, and TWEEN 80.

The components and the weight percentage concentrations of Embodiment A4 to Embodiment A6 were substantially similar to those of Embodiment A2. The differences were that Embodiment A4 to Embodiment A6 included both zinc sulfate and zinc lactate. In Embodiment A4 to Embodiment A6, zinc sulfate and zinc lactate in the liquid composition had the weight percentage concentration of 0.001%, 0.25%, and 1%, respectively.

The components and the weight percentage concentrations of Embodiment A7 to Embodiment A8 were substantially similar to those of Embodiment A5. The difference was that Embodiment A7 further included vitamin B6 and vitamin B12, and Embodiment A8 further included vitamin B6, vitamin B12, and vitamin E.

The component and the weight percentage concentration of Embodiment A9 were substantially similar to those of Embodiment A6. The difference was that Embodiment A9 further included vitamin B6, vitamin B12, and vitamin E.

The components and the weight percentage concentrations of Embodiment A10 to Embodiment A12 were substantially similar to those of Embodiment A8. The difference was that Embodiment A10 did not include hyaluronic acid, Embodiment A11 did not include hyaluronic acid and PEG 400, and Embodiment A12 did not include hyaluronic acid, PEG 400, and TWEEN 80.

The comparative embodiments included Comparative Embodiment B1 to Comparative Embodiment B3. The components and the weight percentage concentrations of Comparative Embodiment B1, Comparative Embodiment B2, and Comparative Embodiment B3 were substantially similar to those of Embodiment A1, Embodiment A2, and Embodiment A8, respectively. The differences were that Comparative Embodiment B1 to Comparative Embodiment B3 did not have any zinc sulfate or zinc lactate. Table 2 shows the specific components of Comparative Embodiment B1 to Comparative Embodiment B3 and their weight percentage concentrations (unit in %) in the liquid composition.

**Table 1**

| | Embodiments | | | | | | | | | | | |
|---|---|---|---|---|---|---|---|---|---|---|---|---|
| | A1 | A2 | A3 | A4 | A5 | A6 | A7 | A8 | A9 | A10 | A11 | A12 |
| Sodium Chloride | 0.69% | | | | | | | | | | | |
| Boric Acid | 0.46% | | | | | | | | | | | |
| Borax | 0.05% | | | | | | | | | | | |
| Hyaluronic Acid | - | 0.05% | | | | | | | | - | 0.05% | - |
| PEG 400 | - | 0.03% | | | | | | | | 0.03% | - | - |
| TWEEN 80 | - | 0.08% | | | | | | | | 0.08% | - | - |
| Zinc Sulfate | 0.001% | 0.001% | - | 0.001% | 0.25% | 1.00% | 0.25% | 0.25% | 1.00% | 0.25% | 0.25% | 0.25% |
| Zinc Lactate | - | - | 0.001% | 0.001% | 0.25% | 1.00% | 0.25% | 0.25% | 1.00% | 0.25% | 0.25% | 0.25% |
| Vitamin B6 | - | - | - | - | - | - | 1.00% | 1.00% | 1.00% | 1.00% | 1.00% | 1.00% |
| Vitamin B12 | - | - | - | - | - | - | 1.50% | 1.50% | 1.50% | 1.50% | 1.50% | 1.50% |
| Vitamin E | - | - | - | - | - | - | - | 0.08% | 0.08% | 0.08% | 0.08% | 0.08% |

**Table 2**

| | Comparative Embodiments | | |
|---|---|---|---|
| | B1 | B2 | B3 |
| Sodium Chloride | 0.69% | | |
| Boric Acid | 0.46% | | |
| Borax | 0.05% | | |
| Hyaluronic Acid | - | 0.05% | |
| PEG 400 | - | 0.03% | |
| TWEEN 80 | - | 0.08% | |
| Zinc Sulfate | - | - | - |
| Zinc Lactate | - | - | - |
| Vitamin B6 | - | - | 1.00% |
| Vitamin B12 | - | - | 1.50% |
| Vitamin E | - | - | 0.08% |

The following was based on the visual analogue scale (VAS) commonly used in the art to measure the evaluation of self-sensory symptoms after wearing contact lenses stored in the liquid compositions of Table 1 (Embodiment A1 to Embodiment A12) and Table 2 (Comparative Embodiment B1 to Comparative Embodiment B3). The evaluation included comfort, water retention and lubrication, visual acuity, and no foreign body sensation. Each evaluation could be divided into the evaluations when subjects were just wearing contact lenses and after 1 hour, 4 hours, and 8 hours of wearing. Self-sensory symptoms were evaluated on a scale of 1 to 10, with higher values indicating better self-sensory symptoms and lower values indicating worse self-sensory symptoms. Table 3 shows the evaluation results of the subject's self-sensory symptoms after wearing contact lenses.

**Table 3**

| VAS Method | | Self-Sensory Symptoms Scores | | | | | | | | | | | | | | |
|---|---|---|---|---|---|---|---|---|---|---|---|---|---|---|---|---|
| | | Embodiments | | | | | | | | | | | | Comparative Embodiments | | |
| Evaluation | Time | A1 | A2 | A3 | A4 | A5 | A6 | A7 | A8 | A9 | A10 | A11 | A12 | B1 | B2 | B3 |
| Comfort | Just Wearing | 7 | 8 | 8 | 8 | 8 | 8 | 8 | 8 | 8 | 8 | 8 | 8 | 6 | 7 | 8 |
| | 1 Hour Wearing | 7 | 8 | 8 | 8 | 8 | 8 | 8 | 8 | 8 | 8 | 8 | 8 | 6 | 7 | 8 |
| | 4 Hours Wearing | 5 | 6 | 6 | 6 | 7 | 8 | 7 | 7 | 8 | 6 | 6 | 6 | 5 | 6 | 7 |
| | 8 Hours Wearing | 4 | 4 | 4 | 4 | 5 | 6 | 6 | 7 | 8 | 5 | 4 | 4 | 4 | 4 | 4 |
| Water Retention and Lubrication | Just Wearing | 6 | 8 | 8 | 8 | 8 | 8 | 8 | 8 | 8 | 8 | 8 | 8 | 6 | 7 | 7 |
| | 1 Hour Wearing | 6 | 8 | 8 | 8 | 8 | 8 | 8 | 8 | 8 | 8 | 7 | 7 | 6 | 7 | 7 |
| | 4 Hours Wearing | 5 | 6 | 6 | 6 | 7 | 8 | 7 | 7 | 8 | 6 | 7 | 6 | 4 | 6 | 7 |
| | 8 Hours Wearing | 4 | 4 | 4 | 4 | 5 | 6 | 5 | 7 | 8 | 5 | 6 | 5 | 4 | 4 | 6 |
| Visual Acuity | Just Wearing | 7 | 8 | 8 | 8 | 8 | 8 | 8 | 8 | 8 | 8 | 8 | 8 | 6 | 7 | 7 |
| | 1 Hour Wearing | 7 | 7 | 7 | 7 | 8 | 8 | 8 | 8 | 8 | 8 | 8 | 8 | 6 | 6 | 7 |
| | 4 Hours Wearing | 5 | 6 | 6 | 6 | 7 | 8 | 8 | 8 | 8 | 8 | 7 | 7 | 5 | 6 | 7 |
| | 8 Hours Wearing | 5 | 6 | 6 | 6 | 7 | 8 | 7 | 7 | 8 | 7 | 7 | 7 | 5 | 6 | 6 |
| No Foreign Body Sensation | Just Wearing | 6 | 8 | 8 | 8 | 8 | 8 | 8 | 8 | 8 | 8 | 8 | 8 | 6 | 7 | 7 |
| | 1 Hour Wearing | 6 | 8 | 8 | 8 | 8 | 8 | 8 | 8 | 8 | 8 | 8 | 8 | 6 | 7 | 7 |
| | 4 Hours Wearing | 6 | 6 | 6 | 6 | 7 | 8 | 7 | 7 | 8 | 7 | 7 | 7 | 5 | 6 | 7 |
| | 8 Hours Wearing | 4 | 4 | 4 | 4 | 5 | 6 | 6 | 6 | 7 | 5 | 6 | 5 | 4 | 4 | 6 |

As can be seen from Table 3, compared with Comparative Embodiment B1, Embodiment A1 has higher self-sensory symptoms evaluation scores in comfort, water retention and lubrication, visual acuity, and no foreign body sensation. It can also be seen from Table 3 that regardless of whether the subject is just wearing the contact lenses, or 1 hour, 4 hours, and 8 hours of wearing, the above self-sensory symptoms evaluation scores in Embodiment A1 are still higher than those of Comparative Embodiment B1. Similarly, the above self-sensory symptoms evaluation scores in Embodiment A2 and Embodiment A8 are also higher than those of Comparative Embodiment B2 and Comparative Embodiment B3, respectively.

It is worth noting that whether it is Embodiment A1 to Embodiment A3 (including one of zinc sulfate or zinc lactate), Embodiment A4 to Embodiment A6 (including both zinc sulfate and zinc lactate), Embodiment A7 (in addition to zinc sulfate and zinc lactate, also including vitamin B6 and vitamin B12), or Embodiment A8 (in addition to zinc sulfate and zinc lactate, also including vitamin B6, vitamin B12, and vitamin E), the evaluation of self-sensory symptoms of the subjects in comfort, water retention and lubrication, visual acuity, and no foreign body sensation are also significantly better than those of Comparative Embodiments B1 to B3.

Accordingly, the present disclosure provides a novel liquid composition for the preservation of contact lenses by adding a specific content of zinc salt and optionally adding certain vitamin species so that subjects can obtain better comfort, water retention and lubrication, visual acuity, and no foreign body sensation when wearing contact lenses.

It is also worth noting that in the absence of hyaluronic acid, PEG 400 and/or TWEEN 80 (see Embodiment A10 to Embodiment A12), the evaluation of the subject's self-sensory symptoms is also significantly better than that of Comparative Embodiment B1 to Comparative Embodiment B3. In the absence of hydrophilic substance and/or a surfactant, the novel liquid composition of the present disclosure for the preservation of contact lenses can also enable subjects to obtain better comfort, water retention and lubrication, visual acuity, and no foreign body sensation when wearing contact lenses.

The second set of embodiments included Embodiment C1 to Embodiment C12. The specific components of Embodiment C1 to Embodiment C12 were substantially individually similar to those of Embodiment A1 to Embodiment A12. The differences were that Embodiment C1 to Embodiment C12 did not include zinc sulfate and/or zinc lactate, but flavin mononucleotide and/or flavin adenine dinucleotide. The concentrations of flavin mononucleotide and/or flavin adenine dinucleotide in the liquid composition were changed. Those skilled in the art should understand that flavin mononucleotide and flavin adenine dinucleotide were some examples of vitamin B2 derivatives. Table 4 records the specific components and the weight percentage concentrations (unit in %) of the liquid composition in Embodiment C1 to Embodiment C12.

Also, according to the visual analogue scale method commonly used in the art to measure, the evaluation of self-sensory symptoms of 5 subjects after wearing contact lenses stored in the liquid composition is shown in Table 4. The specific content of the evaluation has been described above and will not be repeated herein. Table 5 shows the results of the evaluation of the subject's self-sensory symptoms after wearing contact lenses.

As can be seen from Table 5, compared with Comparative Embodiment B1, Embodiment C1 has higher self-sensory symptoms evaluation scores in comfort, water retention and lubrication, visual acuity, and no foreign body sensation. It can also be seen from Table 5 that regardless of whether the subject is just after wearing contact lenses or after 1 hour, 4 hours, and 8 hours of wearing, in the evaluation score of the above self-sensory symptoms, Embodiment C1 is still higher than the Comparative Embodiment B1. Similarly, the above self-sensory symptoms evaluation scores of Embodiment C2 and Embodiment C8 are also higher than those of Comparative Embodiment B2 and Comparative Embodiment B3, respectively.

It is worth noting that whether it is Embodiment C1 to Embodiment C3 (including flavin mononucleotide or flavin adenine dinucleotide), Embodiment C4 to Embodiment C6 (including flavin mononucleotide and flavin adenine dinucleotide), Embodiment C7 (including flavin mononucleotide and flavin adenine dinucleotide, in addition to vitamin B6 and vitamin B12), or Embodiment C8 (including flavin mononucleotide and flavin adenine dinucleotide, in addition to vitamin B6, vitamin B12, and vitamin E), the subjects' evaluation of self-sensory symptoms in comfort, water retention and lubrication, visual acuity, and no foreign body sensation are also significantly better than those of Comparative Embodiment B1 to Comparative Embodiment B3. Accordingly, it can be seen that the present disclosure provides a novel liquid composition for the preservation of contact lenses by adding a specific content of flavin mononucleotide and/or flavin adenine dinucleotide and optionally adding certain other vitamins so that subjects can obtain better comfort, water retention and lubrication, visual acuity, and no foreign body sensation when wearing contact lenses.

Further, in the absence of hyaluronic acid, PEG 400 and/or TWEEN 80 (see Embodiment C10 to Embodiment C12), the evaluation of the subject's self-sensory symptoms are also significantly better than those of Comparative Embodiment B1 to Comparative Embodiment B3. In the absence of a hydrophilic substance and/or a surfactant, the novel liquid composition of the present disclosure for the preservation of contact lenses can also enable subjects to obtain better comfort, water retention and lubrication, visual acuity and, no foreign body sensation when wearing contact lenses.

**Table 4**

| | Embodiments | | | | | | | | | | | |
|---|---|---|---|---|---|---|---|---|---|---|---|---|
| | C1 | C2 | C3 | C4 | C5 | C6 | C7 | C8 | C9 | C10 | C11 | C12 |
| Sodium Chloride | 0.69% | | | | | | | | | | | |
| Boric Acid | 0.46% | | | | | | | | | | | |
| Borax | 0.05% | | | | | | | | | | | |
| Hyaluronic Acid | - | 0.05% | | | | | | | | - | 0.05% | - |
| PEG 400 | - | 0.03% | | | | | | | | 0.03% | - | - |
| TWEEN 80 | - | 0.08% | | | | | | | | 0.08% | - | - |
| Flavin Mononucleotide | - | - | 0.03% | 0.03% | 0.05% | 0.30% | 0.05% | 0.05% | 0.30% | 0.05% | 0.05% | 0.05% |
| Flavin Adenine Dinucleotide | 0.0001% | 0.03% | | 0.03% | 0.05% | 0.30% | 0.05% | 0.05% | 0.30% | 0.05% | 0.05% | 0.05% |
| Vitamin B6 | - | - | - | - | - | - | 1.00% | 1.00% | 1.00% | 1.00% | 1.00% | 1.00% |
| Vitamin B12 | - | - | - | - | - | - | 1.50% | 1.50% | 1.50% | 1.50% | 1.50% | 1.50% |
| Vitamin E | - | - | - | - | - | - | - | 0.08% | 0.08% | 0.08% | 0.08% | 0.08% |

**Table 5**

| VAS Method | | Self-Sensory Symptoms Scores | | | | | | | | | | | | | | |
|---|---|---|---|---|---|---|---|---|---|---|---|---|---|---|---|---|
| | | Embodiments | | | | | | | | | | | | Comparative Embodiments | | |
| Evaluation | Time | C1 | C2 | C3 | C4 | C5 | C6 | C7 | C8 | C9 | C10 | C11 | C12 | B1 | B2 | B3 |
| Comfort | Just Wearing | 6 | 7 | 7 | 8 | 9 | 9 | 9 | 9 | 9 | 8 | 8 | 8 | 6 | 7 | 8 |
| | 1 Hour Wearing | 6 | 7 | 7 | 8 | 9 | 9 | 9 | 9 | 9 | 8 | 8 | 8 | 6 | 7 | 8 |
| | 4 Hours Wearing | 6 | 7 | 7 | 7 | 7 | 8 | 8 | 8 | 8 | 7 | 6 | 6 | 5 | 6 | 7 |
| | 8 Hours Wearing | 4 | 4 | 4 | 4 | 4 | 7 | 5 | 5 | 7 | 5 | 5 | 5 | 4 | 4 | 4 |
| Water Retention and Lubrication | Just Wearing | 6 | 7 | 7 | 8 | 9 | 9 | 9 | 9 | 9 | 8 | 8 | 8 | 6 | 7 | 7 |
| | 1 Hour Wearing | 6 | 7 | 7 | 8 | 9 | 9 | 9 | 9 | 9 | 8 | 7 | 7 | 6 | 7 | 7 |
| | 4 Hours Wearing | 5 | 7 | 7 | 7 | 7 | 8 | 8 | 8 | 8 | 7 | 7 | 7 | 4 | 6 | 7 |
| | 8 Hours Wearing | 4 | 4 | 4 | 4 | 5 | 6 | 5 | 6 | 7 | 6 | 5 | 5 | 4 | 4 | 6 |
| Visual Acuity | Just Wearing | 6 | 7 | 7 | 8 | 9 | 9 | 9 | 9 | 9 | 9 | 8 | 8 | 6 | 7 | 7 |
| | 1 Hour Wearing | 6 | 7 | 7 | 8 | 9 | 9 | 9 | 9 | 9 | 9 | 8 | 8 | 6 | 6 | 7 |
| | 4 Hours Wearing | 6 | 7 | 7 | 7 | 7 | 8 | 8 | 8 | 8 | 8 | 8 | 8 | 5 | 6 | 7 |
| | 8 Hours Wearing | 5 | 6 | 6 | 6 | 7 | 8 | 7 | 7 | 8 | 7 | 7 | 7 | 5 | 6 | 6 |
| No Foreign Body Sensation | Just Wearing | 6 | 7 | 7 | 8 | 9 | 9 | 9 | 9 | 9 | 9 | 8 | 8 | 6 | 7 | 7 |
| | 1 Hour Wearing | 6 | 7 | 7 | 8 | 9 | 9 | 9 | 9 | 9 | 9 | 8 | 8 | 6 | 7 | 7 |
| | 4 Hours Wearing | 6 | 7 | 7 | 7 | 7 | 8 | 7 | 7 | 8 | 7 | 7 | 7 | 5 | 6 | 7 |
| | 8 Hours Wearing | 4 | 4 | 4 | 4 | 5 | 6 | 5 | 5 | 7 | 5 | 5 | 5 | 4 | 4 | 6 |

The third set of embodiments included Embodiment D1 to Embodiment D4. Table 6 shows the specific components and the weight percentage concentrations (unit in %) of Embodiment D1 to Embodiment D4 in the liquid composition. As shown in Table 6, each Embodiment D1 to Embodiment D4 includes 0.20% zinc sulfate, 0.05% zinc lactate, and 0.05% flavin mononucleotide, while Comparative Embodiment B1 to Comparative Embodiment B3 is without any one of zinc sulfate, zinc lactate, and flavin mononucleotide.

Also, according to the visual analogue scale method commonly used in the art, the evaluation of self-sensory symptoms of 5 subjects after wearing contact lenses stored in the liquid composition is shown in Table 6. The specific content of the evaluation has been described above and will not be repeated herein. Table 7 shows the results of the evaluation of the subjects' self-sensory symptoms after wearing contact lenses.

As can be seen from Table 7, compared with Comparative Embodiment B1 to Comparative Embodiment B3, Embodiment D1 to Embodiment D4 have higher self-sensory symptoms evaluation scores in comfort, water retention and lubrication, visual acuity, and no foreign body sensation. In other words, in the case of simultaneously including zinc sulfate, zinc lactate, flavin mononucleotide and, optionally the addition of certain kinds of vitamins, the novel liquid composition of the present disclosure for the preservation of contact lenses can enable subjects to obtain better comfort, water retention and lubrication, visual acuity, and no foreign body sensation when wearing contact lenses. Further, Embodiment D1 also shows that in the absence of hyaluronic acid and PEG 400, the evaluation of the subjects' self-sensory symptoms are also significantly better than those of Comparative Embodiment B1 to Comparative Embodiment B3.

**Table 6**

| | Embodiments | | | |
|---|---|---|---|---|
| | D1 | D2 | D3 | D4 |
| | 0.69% | 0.69% | 0.69% | 0.69% |
| Boric Acid | 0.46% | 0.46% | 0.46% | 0.46% |
| Borax | 0.05% | 0.05% | 0.05% | 0.05% |
| Hyaluronic Acid | - | 0.05% | 0.05% | 0.05% |
| PEG 400 | - | 0.03% | 0.03% | 0.03% |
| TWEEN 80 | - | - | 0.08% | 0.08% |
| Zinc Sulfate | 0.20% | 0.20% | 0.20% | 0.20% |
| Zinc Lactate | 0.05% | 0.05% | 0.05% | 0.05% |
| Flavin Mononucleotide | 0.05% | 0.05% | 0.05% | 0.05% |
| Flavin Adenine Dinucleotide | - | - | - | - |
| Vitamin B6 | - | - | - | 1.00% |
| Vitamin B12 | - | - | - | 1.50% |
| Vitamin E | - | - | - | 0.08% |

**Table 7**

| VAS Method | | Self-Sensory Symptoms Scores | | | | | | |
|---|---|---|---|---|---|---|---|---|
| | | Embodiments | | | | Comparative Embodiments | | |
| Evaluation | Time | D1 | D2 | D3 | D4 | B1 | B2 | B3 |
| Comfort | Just Wearing | 8 | 9 | 9 | 9 | 6 | 7 | 8 |
| | 1 Hour Wearing | 8 | 9 | 9 | 9 | 6 | 7 | 8 |
| | 4 Hours Wearing | 7 | 7 | 8 | 8 | 5 | 6 | 7 |
| | 8 Hours Wearing | 5 | 5 | 5 | 7 | 4 | 4 | 4 |
| Water Retention and Lubrication | Just Wearing | 8 | 9 | 9 | 9 | 6 | 7 | 7 |
| | 1 Hour Wearing | 8 | 9 | 9 | 9 | 6 | 7 | 7 |
| | 4 Hours Wearing | 7 | 7 | 8 | 8 | 4 | 6 | 7 |
| | 8 Hours Wearing | 5 | 5 | 5 | 7 | 4 | 4 | 6 |
| Visual Acuity | Just Wearing | 9 | 9 | 9 | 9 | 6 | 7 | 7 |
| | 1 Hour Wearing | 8 | 9 | 9 | 9 | 6 | 6 | 7 |
| | 4 Hours Wearing | 7 | 7 | 8 | 8 | 5 | 6 | 7 |
| | 8 Hours Wearing | 7 | 7 | 7 | 7 | 5 | 6 | 6 |
| No Foreign Body Sensation | Just Wearing | 8 | 9 | 9 | 9 | 6 | 7 | 7 |
| | 1 Hour Wearing | 8 | 9 | 9 | 9 | 6 | 7 | 7 |
| | 4 Hours Wearing | 7 | 7 | 8 | 8 | 5 | 6 | 7 |
| | 8 Hours Wearing | 5 | 5 | 5 | 7 | 4 | 4 | 6 |

In summary, this disclosure provides a liquid composition for ophthalmic products, such as a liquid composition for the preservation of contact lenses. The liquid composition includes a specific addition amount of zinc salt, vitamin B2 and/or vitamin B2 derivative. The above zinc salt can release zinc ions in a buffer solution of the liquid composition. Many experiments had proved that when the subject wore contact lenses pre-stored in the liquid composition of this disclosure, the subject could obtain better comfort, water retention and lubrication, visual acuity, and no foreign body sensation. Moreover, the subject could still have good comfort, water retention and lubrication, visual acuity, and no foreign body sensation after just wearing contact lenses, or after 1 hour, 4 hours, and 8 hours of wearing. In other words, by providing a novel liquid composition to preserve contact lenses, the disclosure could effectively improve the subjects' feeling of wearing contact lenses, helping to maintain the subjects' cornea health.

## Claims

1. A liquid composition for ophthalmic products, **characterized by** comprising:
a buffer solution; and
one or more selected from the group consisting of a zinc salt and a first vitamin, wherein:
the zinc salt is dissolved in the buffer solution and has a weight percentage concentration of 1×10⁻⁵% to 3% in the liquid composition; and
the first vitamin is dissolved in the buffer solution and has a weight percentage concentration of 1×10⁻⁵% to 1 % in the liquid composition, and the first vitamin comprises vitamin B2, vitamin B2 derivative, or a combination thereof.

2. The liquid composition of claim 1, **characterized in that** the weight percentage concentration of the zinc salt is between 5×10⁻⁵% to 2%.

3. The liquid composition of any one of claims 1 to 2, **characterized in that** the zinc salt comprises zinc sulfate, zinc sulfate hydrate, zinc gluconate, zinc lactate, zinc chloride, zinc citrate, chelated zinc, zinc acetate, or combinations thereof.

4. The liquid composition of any one of claims 1 to 3, **characterized in that** the weight percentage concentration of the first vitamin is between 5×10⁻⁵% to 0.5%.

5. The liquid composition of any one of claims 1 to 4, **characterized in that** the first vitamin comprises riboflavin, flavin mononucleotide and its salt, flavin adenine dinucleotide and its salt, or combinations thereof.

6. The liquid composition of any one of claims 1 to 5, **characterized in that** the buffer solution comprises a borate, a phosphate, or a combination thereof.

7. The liquid composition of claim 6, **characterized in that** the buffer solution further comprises a boric acid, a sodium chloride, or a combination thereof.

8. The liquid composition of any one of claims 6 to 7, **characterized by** further comprising a hydrophilic substance, a surfactant, a second vitamin, or combinations thereof.

9. The liquid composition of claim 8, **characterized in that** the second vitamin comprises vitamin A, vitamin A derivative, vitamin B3, vitamin B3 derivative, vitamin B6, vitamin B6 derivative, vitamin B12, vitamin B12 derivative, vitamin C, vitamin C derivative, vitamin E, vitamin E derivative, or combinations thereof.

10. The liquid composition of any one of claims 8 to 9, **characterized in that** the second vitamin has a weight percentage concentration of 1×10⁻⁵% to 5% in the liquid composition.

11. The liquid composition of claim 10, **characterized in that** the weight percentage concentration of the second vitamin is between 5×10⁻⁵% to 3%.

12. The liquid composition of any one of claims 10 to 11, **characterized in that** the hydrophilic substance has a weight percentage concentration of 1×10⁻³% to 3% in the liquid composition.

13. The liquid composition of claim 12, **characterized in that** the weight percentage concentration of the hydrophilic substance is between 5×10⁻³% to 2.5%.

14. The liquid composition of any one of claims 12 to 13, **characterized in that** the surfactant has a weight percentage concentration of 1×10⁻³% to 2% in the liquid composition.

15. The liquid composition of claim 14, **characterized in that** the weight percentage concentration of the surfactant is between 5×10⁻³% to 1%.
